# EUROPEAN PATENT SPECIFICATION

(11) **EP 2 316 519 B1**
(45) Date of publication and mention of the grant of the patent: **05.09.2012**
(21) Application number: 10251829.7
(22) Date of filing: 21.10.2010
(51) Int. Cl.: A61M 25/09

(54) **A medical guide wire, a method of making the same, an assembly of microcatheter and guiding catheter combined with the medical guide wire**
Medizinischer Führungsdraht, Verfahren zu dessen Herstellung und Anordnung eines Mikrokatheters und Kombination aus Führungskatheter und dem medizinischen Führungsdraht
Fil-guide médical, son procédé de fabrication, assemblage de microcathéter et cathéter de guidage combiné au fil-guide médical

(30) Priority: 27.10.2009 JP 2009246937
(43) Date of publication of application: 04.05.2011
(73) Proprietor: PatentStra Co. Ltd., Anjo-shi Aichi 446-0008 (JP)
(72) Inventor: Kato, Tomihisa, Aichi 446-0008 (JP)
(74) Representative: Hopkin, Tobias J.B.

(56) References cited:
- EP-A1- 1 685 869
- EP-A1- 1 803 483
- US-A1- 2004 167 441
- US-A1- 2005 027 214
- US-A1- 2008 171 217

## Description

The present invention relates to a method of making a medical guidewire which improves a mechanical strength properties of a welded portion upon welding a core wire and a helical spring body at their distal end tips by means of a welding member.

In general, a medical guide wire (referred to simply as a guide wire) is thinned so that the guide wire is inserted into a somatic vasculature. With the thinned wire in mind, it is necessary to impart mechanical requirements to the guide wire with safety measures secured for a human body. For this purpose, various types of contrivances have been introduced. Such a method is disclosed in the US 2008171217.

In Japanese Laid-open Patent Application No. 2003-164530 (referred to as first reference), the first reference discloses a medical guide wire in which a head plug is determined at its lengthwise dimension to make the head plug pass through a diseased area of the vasculature so as to therapeutically treat a stenosed lesion.

The first reference, however, remains silent about a welding structure which improves its mechanical strength properties between a core wire and a helical spring body in a tangible terms without reducing the mechanical strength properties.

In Japanese Laid-open Patent Application No. 2003-135603 (referred to as second reference), the second reference discloses a medical guide wire in which the core wire projects its distal end portion exterior out of a helical spring body in order not to sacrifice the metallurgical properties due to the thermal influence when soldering the core wire to the helical spring body.

The second reference, however, remains silent about grasping the metallurgical properties of the core wire and the heating temperature of the core wire when the core wire is subjected to the heat upon soldering the core wire to the helical spring body, so as to improve the mechanical strength properties of a welded portion (head plug) between the core wire and the helical spring body without sacrificing the mechanical strength properties. Still less, the second reference discloses no tangible means to lengthwisely reduce or diametrically minimize the head plug.

In Japanese Laid-open Patent Application No. 44-18710 (referred to as third reference), the third reference discloses a helical spring guide, a part of helices of which is squelched flat so as to enable an operator to arcuately bend or bend back straight.

Although the third reference teaches that the wire core welds its distal end tip to the helical spring guide by way of a front cap, no tangible means is disclosed to improve the mechanical strength properties of the welded portion between the core wire and the helical spring guide by utilizing the melting heat produced upon soldering the core wire to the helical spring guide.

In Japanese Laid-open Patent Application No. 2005-6868 (referred to as fourth reference), the fourth reference discloses a medical guide wire which provides a core wire with a bulged portion so as to increase its cross sectional area. This prevents the core wire from reducing its mechanical strength properties due to the thermal influence (annealing) upon welding the core wire to a helical spring body.

Although the fourth reference shows that a head plug measures 1.0mm along its lengthwise direction, it teaches no tangible means to shorten and diametrically minimize the head plug. Let alone, the fourth reference shows no concrete measure to improve the mechanical strength properties of the welded portion between the core wire and the helical spring body by utilizing the melting heat produced upon soldering the core wire to the helical spring body.

In the prior medical guide wires, no technological idea has been introduced that a core wire (stainless steel wire) is highly drawn by means of a tightly drawing procedure to produce a highly drawn core wire, and a eutectic alloy is used as a welding member (soldering or brazing material) with the thermal influence against the mechanical strength properties taken into consideration upon forming a head plug by welding the core wire to a helical spring body.

Further, no technological idea has been introduced so far to lengthwisely reduce and diametrically minimize the head plug to enable the operator to deeply insert the head plug readily into a sinuous or minutely meandering path of the blood vessel.

Therefore, the present invention has been made with the above drawbacks in mind, it is a main object of the invention to provide a method of making a guidwire wich uses an austenitic stainless steel wire highly drawn as a core wire to improve the mechanical strength properties by utilizing the thermal influence given to the core wire when subjected to the melting heat upon welding the core wire to a helical spring body without sacrificing the mechanical strength properties due to the thermal influence, thus making it possible to tightly weld a head plug between the core wire and the helical spring body.

It is another object of the invention to provide a method of making a medical guidewire which is capable to improve the mechanical strength and the wear-resistant property of the welded portion at the head plug so as to lengthwisely reduce and diametrically minimize the head plug to enable an operator to deeply insert the head plug readily into a sinuous or minutely meandering path of the blood vessel so as to enable the operator to use it safely.

According to the present invention, there is provided a method of making a medical guide wire as further disclosed in claim 1 having a core wire formed by a flexible elongate member, a helical spring body inserted to a distal end portion of the core wire to be placed around the core wire, and a head plug provided at distal end tips of both the core wire and the helical spring body by means of a welding member.

Drawn is the core wire which is made of an austenitic stainless steel wire treated with a solid solution procedure until a whole cross sectional reduction ratio of the core wire reaches 90%-99%. The distal end portion of the core wire is ground. The helical spring body is assembled by inserting the helical spring body to the distal end portion of the core wire to be placed around the core wire. A weld-hardened portion is formed at a welded portion between the distal end tips of the core wire and the helical spring body by melting a welding member as a eutectic alloy having a melting temperature of 180°C -995°C including a eutectic alloy having a melting temperature of 180°C -525°C when the austenitic stainless steel wire of the core wire has molybdenum as a component. A minor weld-hardened portion is formed by severing the weld-hardened portion at a predetermined length from a distal tip portion of the weld-hardened portion. A head-most portion is provided by the same or same type of a welding member from which the minor weld-hardened portion is made, and attaching the head-most portion in integral with a distal tip portion of the minor weld-hardened portion so as to form the head plug.

With the method as mentioned above, it is possible to make a good use of the welding heat of the welding member so as increase the tensile rupture strength of the core wire, and synergistically increasing the welding strength of the core wire at a welded portion (head plug) between the core wire and the helical spring body with the austenitic stainless steel wire highly drawn as the core wire, thus making it possible to lengthwisely reduce and diametrically minimize the head plug with a good maneuverability imparted to the core wire.

According to other aspect of the present invention, after grinding the distal end portion of the core wire, a distal end of the core wire is heat treated partially at a temperature of 180°C - 495°C at least at a portion in which the head plug is formed, including a temperature of 180°C -525°C when the austenitic stainless steel wire of the core wire has molybdenum as a component element. A welding member is molten to be poured over an outer surface of the core wire by a predetermined length thereof so as to form a film layer, so that the distal end of the core wire, the minor weld-hardened portion and the head-most portion are united integrally to form the head plug. The welding member is the same or same type of a welding member as used when the head plug is formed.

With the above method, it becomes possible to increase the tensile rupture strength of the core wire by forming it from highly drawn austenitic stainless steel wire, while at the same time, improving the wetting property of the core wire against the welding member, and increasing the unified strength (welding strength) of the head plug by uniting the minor weld-hardened portion and the head-most portion together. This contributes to lengthwisely reduce and diametrically minimize the head plug.

A preferred form of the present invention is illustrated in the accompanying drawings in which:
Fig. 1 is a longitudinal cross sectional view of a medical guide wire according to a first embodiment of the invention;
Fig. 2 is a right side elevational view of the medical guide wire;
Fig. 3 is a plan view of a core wire;
Fig. 4 is a right side elevational view of the core wire;
Fig. 5 is a longitudinal cross sectional view showing a distal end portion of the medical guide wire
Fig. 6 is a left side elevational view of the medical guide wire;
Figs. 7, 8 and 9 are perspective views each showing a distal end portion of the core wire;
Figs. 10 and 11 are sequential processes (A)-(F), (a)-(d) showing how to weld the distal end of the core wire to a helical spring body;
Fig. 12 is a graphical representation showing a relationship between a length of a head plug and a break-away strength;
Fig. 13 is a graphical representation of a tensile strength characteristics showing a relationship between a temperature and a tensile rupture strength;
Fig. 14 is a graphical representation of a tensile strength characteristics showing a relationship between a whole cross sectional reduction ratio and a tensile rupture strength;
Figs. 15, 16 are schematic views showing how to insert the medical guide wire into a completely occluded lesion of the cardiovascular system according to a second embodiment of the invention;
Fig. 17 is a longitudinal cross sectional view of a preshaped configuration represented at the distal end portion of the medical guide wire;
Fig. 18 is a longitudinal cross sectional view of a distal end portion of a microcatheter according to a third embodiment of the invention;
Fig. 19 is a longitudinal cross sectional view of a distal end portion of a prior art medical guide wire; and
Fig. 20 is a left side elevational view of the prior art medical guide wire.

In the following description of the depicted embodiments, the same reference numerals are used for features of the same type.

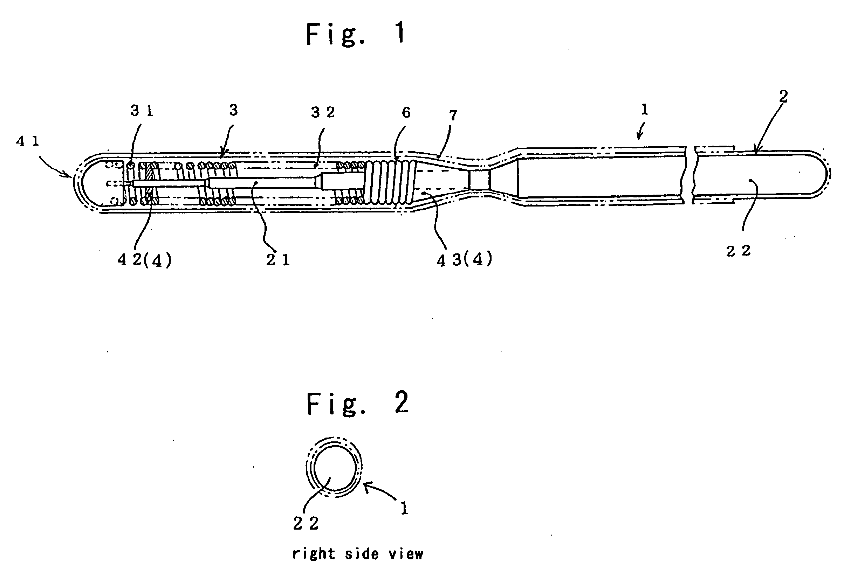

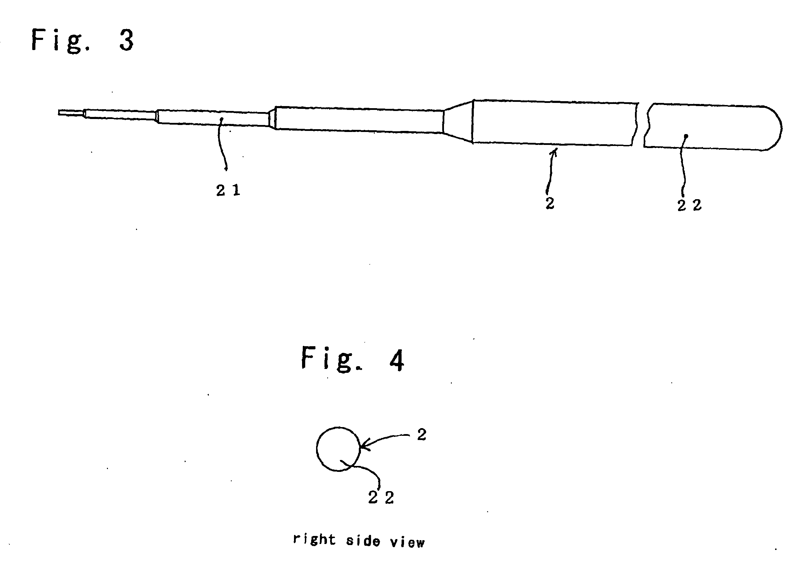

Referring to Figs. 1 through 14 which show a medical guide wire 1 (referred to simply as "a guide wire 1" hereinafter) according to a first embodiment of the invention.

The guide wire 1 has a core wire 2 formed by a flexible elongate member. The core wire 2 has a distal end portion 21 , around which a helical spring body 3 is coaxially placed as shown in Figs. 1 through 4.

The helical spring body 3 has a distal end portion as a radiopaque coil 31 which is made of silver, platinum, wolfram or the like.

At a front welding section 41, a middle welding section 42 and a rear welding section 43 each designated by the distal end portion 21 of the core wire 2, the core wire 2 and the helical spring body 3 are partly secured by means of a welding member 4.

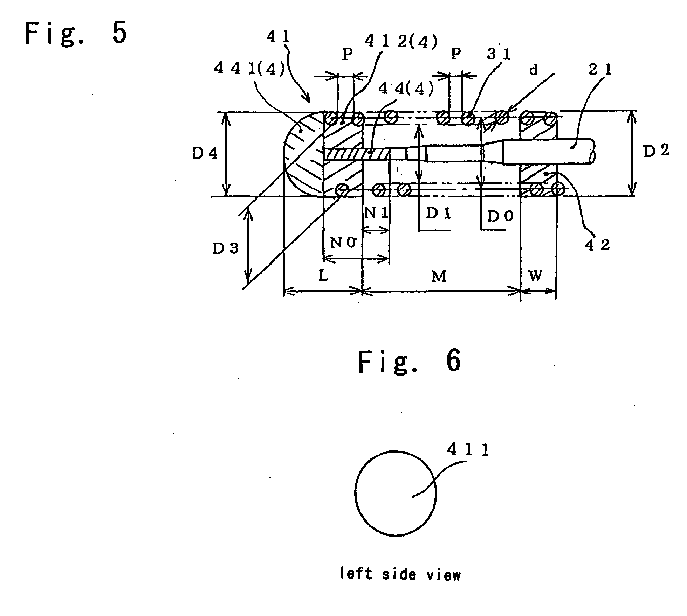

At a distal extremity of the core wire 2, a head plug 41 is provided which is made of the welding member 4 to connectedly secure the spring body 3 to the core wire 2 as shown in Figs. 5, 6.

The core wire 2 has a distal end portion, an outer surface of which is coated with a film layer 44 by means of the welding member 4.

In this instance a minor weld-hardened portion 412 is formed into disc-shaped configuration by severing a weld-hardened portion 413 by a predetermined length from a distal tip portion thereof as described in detail hereinafter.

The minor weld-hardened portion 412 and a convexly curved head-most portion 411 are coaxially arranged and integrally welded together to form the head plug 41 and welded to the distal end portion 21 of the core wire 2 through the film layer 44.

With the use of the welding member 4, the middle welding section 42 is formed between the core wire 2 and the helical spring body 3. It is to be noted that the head-most portion 411 may be formed into a cone-shaped, cylindrical or semi-spherical configuration.

The distal end portion 21 of the core wire 2 which extends by 300mm from its distal end extremity is thinned to measure approximately 0.060mm-0.200mm in diameter. The rest of the core wire 2 corresponds to a proximal portion 22 made of thicker helices extending by approximately 1200mm-2700mm.

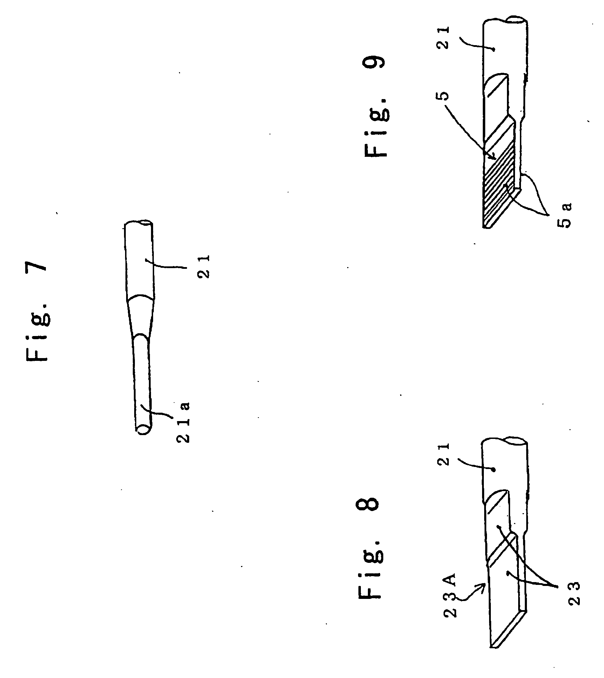

The distal end portion 21 has a diameter-reduced section 21a, a diameter of which decreases progressively as approaching forward as shown in Fig. 7. The diameter-reduced tip section may be circular, square or rectangular in cross section as observed at numerals 21a, 23 and 5 in Figs. 7, 8 and 9.

On an outer surface of the proximal end portion 22 of the core wire 2, coated is a synthetic layer 6 which is made of polyurethane, fluorocarbon resin (e.g., polytetrafluoroethylene (PTFE)) or other polymers. On an outer surface of the spring body 3, coated is a synthetic resin layer which is formed by polyurethane or other polymers. An outer surface of a proximal end portion 22 of the core wire 2 is coated with fluorocarbon resin (e.g., PTFE) or other polymers.

The synthetic layer 6 has an outer surface coated with a hydrophilic polymer 7, an outer diameter of which measures 0.355mm. The hydrophilic polymer 7 works as a lubricant (e.g., polyvinylpyrrolidone) which exhibits the lubricity when moistened.

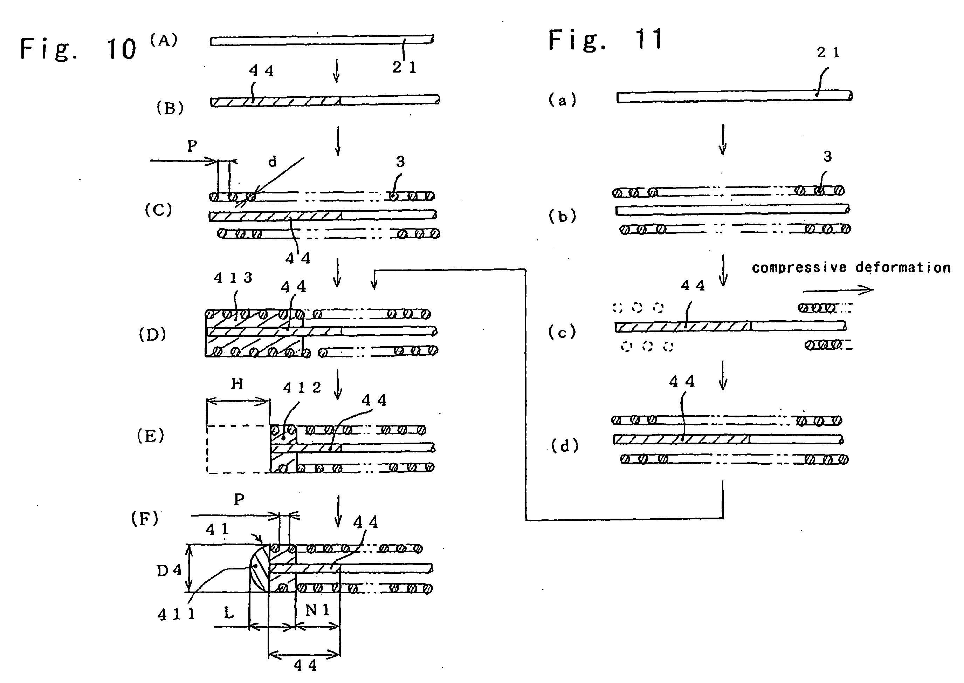

Figs. 10 and 11 are schematic views of sequential processes (A)-(F) and (a)-(d) showing how a head plug 41 is manufactured. At the process (A), prepared is the distal end portion 21 of the core wire 2. On the outer surface of the distal end portion 21, coated is the film layer 44 which is made from the welding member 4 and measures 0.002mm-0.005mm in thickness at the process (B).

At the process (C), the helical spring body 3 is inserted to the core wire 2 to surround the core wire 2, in which a clearance P between helices of the helical spring body 3 is 5%-85% of the wire diameter of the helical spring body 3.

By way of the film layer 44 at the process (D), the weld-hardened portion 413 is formed by welding the helical spring body 3 to the distal end portion 21 of the core wire 2 by means of the welding member 4.

At the process (E), the minor weld-hardened portion 412 is formed into disc-shaped configuration by severing the weld-hardened portion 413 together with the distal end portion 21 by a predetermined length H from the distal end tip of the weld-hardened portion 413.

At the process (F), used is the welding member 4 which has the same or same type of material as the head-most portion 411 and the minor weld-hardened portion 412 in order to integrally weld the head-most portion 411 to a front surface of the minor weld-hardened portion 412 to form the head plug 41 (0.345mm in outer diameter D4).

It is to be noted that the film layer 44 may be formed after inserting the helical spring body 3 to the core wire 2 and proximally pressing the helical spring body 3 to deform in the compressive direction as shown at the process (c) among other processes (a), (b), (d) in Fig. 11.

The clearance P is predetermined to be 5%-85% of the wire diameter of the helical spring body 3. This is because it becomes difficult to permeate (pour) the molten welding member 4 inside the helical spring body 3 through the clearance P when the clearance P is less than 5% of the wire diameter of the helical spring body 3.

When the clearance P exceeds 85% of the wire diameter of the helical spring body 3, it becomes hard to obtain a sufficient contact area between the minor weld-hardened portion 412 and the helicies of the helical spring body 3.

Considering the permeability of the molten welding member 4 and the sufficient contact area with the least length of the helices, it is preferable to determine the clearance to be 5%-65% of the wire diameter of the helical spring body 3. This is all the more true upon considering a break-away strength which needs to come off the head plug 41 from the core wire 2 and the helical spring body 3.

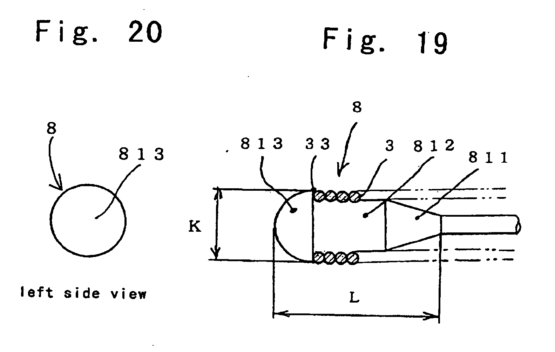

Figs. 19 and 20 show a head plug 8 of a medical guide wire according to Japanese Laid-open Patent Application No. 2005-6868 (fourth reference) as a prior art counterpart (comparative specimen 1).

The core wire 2 has a bulged reinforcement section 811 and a plug base 812, both of which have a cross sectional area larger that that of the core wire 2 in order to attain a sufficient tensile rupture strength. This is due to the reason that the distal end portion 21 of the core wire 2 (wire diameter: 0.06mm) is annealed by the welding heat upon welding the head plug 8 to the distal end portion 21 of the core wire 2. In this instance, the head plug 8 measures 1.0mm in length L with an outer diameter K as 0.345mm.

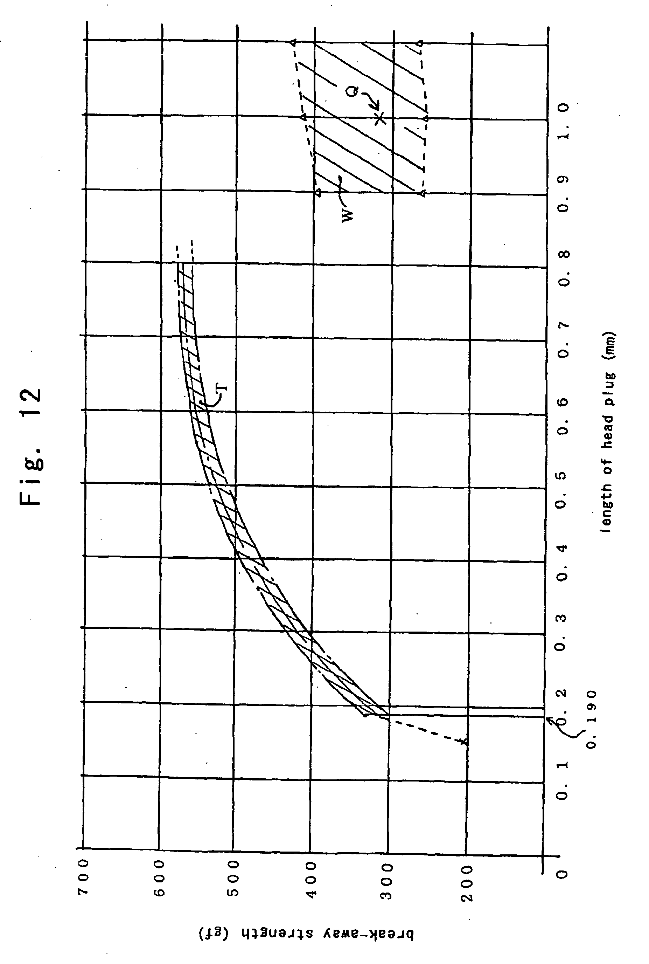

Fig. 12 shows how,the break-away strength changes depending on the length L of the head plug 41 (Fig. 5 and process (F) in Fig. 10). The break-away strength means a maximum load value needed to come off the head plug 41 from the distal end portion 21 of the core wire 2 or helical spring body 3 (radiopaque coil 31 in the first embodiment) by the destruction of the welded portion therebetween when the head plug 41 is subjected to a significant tensile force in the lengthwise direction.

In the guide wire 1, the break-away strength of the head plug 41 is generally determined to be 250gf as a guaranteed lower limit value. For the prior art counterpart (comparative specimen 1), the break-away strength is 320gf on overage which exceeds the lower limit value (250gf) by approximately 70gf, but does not remarkably exceed the average value as shown at Q in Fig. 12.

This is due to the reason that a bulged head 813 is secured to the helix of the helical spring body 3 at the welded portion 33 in a point-to-point contact by means of the TIG welding procedure.

In the comparative specimen 1, the break-away strength depends on the tensile rupture strength and the welding strength of a single helix of the helical spring body 3 against the bulged head 813. The break-away strength is influenced by the welding strength and the melting heat (800°C-900°C) with the head plug 8 determined to be 1.0mm in length (L) as observed in Fig. 19.

Contrary to the comparative specimen 1, the break-away strength exhibits 320gf on average which exceeds the guaranteed lower limit value as shown at T in Fig. 12 when the length L of the head plug 41 is 0.190mm (Fig. 5 and (F) in Fig. 10).

When the length L of the head plug 41 comes to 0.250mm, 0.500mm, 0.600mm and 0.800mm respectively, the break-away strength in turn exhibits 375gf, over 500gf, 550gf and stable 575gf each on average. This makes it possible to reduce the length L of the head plug 41 to be 0.190mm (approximately 1/5) with the break-away strength commonly set as 320g between the comparative specimen 1 and the first embodiment of the invention.

The reasons why the head plug is shortened are described from the view points of the mechanical strength property, the characteristics of the welding member, the welding structure and the head plug structure.

In this instance, a second comparative specimen 2 is introduced in which the welding member is a silver brazing which has 605°C-800°C as the melting temperature, and the head plug is formed at the distal end of the helical spring body by means of the silver brazing with the whole reduction ratio of the core wire 2 as 70% as shown at W in Fig. 12.

The head plug formed in the second comparative specimen 2 has neither a head-most portion nor a minor weld-hardened portion with no film layer, contrary to the head plug 41 of the first embodiment of the invention.

In the second comparative specimen 2, its structure lengthens and hardens the permeating dollop of the molten welding member formed at the time of invading into an annular space between the core wire 2 and the helical spring body 3 by means of the capillary phenomenon.

This makes it difficult to manufacture the head plug less than 0.900mm in length along the lengthwise direction of the core wire, so that values of the break-away strength depicted in Fig. 12 are those when the head plug is 0.900mm or more in length. Hatched areas in Fig. 12 represent a region between an upper limit value and a lower limit value of the break-away strength.

The length (L: 0.190mm) of the head plug 41 in Fig. 5 is a total dimension calculated by three figures below the decimal point. That is the sum value L of two-fold wire diameter (2×0.055mm) of the radiopaque coil 31, the clearance P (5% of the wire diameter) and the length (0.078) of the head-most portion 411.

The above relationship is expressed as follows:
0.078 + 2.05d ≦ L ≦ 0.800 Where L (mm) is the length of the head plug 41, and d (mm) is the wire diameter of the helical spring body 3 under the condition that the length of the head plug 41 is 0.190mm or more. It is preferable to determine that the length L of the head plug 41 is 0.190mm or more but 0.600mm or less.

The length of the head plug 41 is 0.800mm or less because it is possible to maintain approximately 1.8 fold of the break-away strength even if the length of the head plug 41 is lengthwisely reduced by approximately 20% compared to the first specimen 1 and the second specimen 2.

It is to be noted that the length of the head plug is preferably 0.600mm or less because it is possible to maintain approximately 1.7 fold of the break-away strength even if the length of the head plug is lengthwisely reduced by approximately 40% compared to the first specimen 1 and the second specimen 2.

The length of the head plug 41 is 0.190mm or more because the break-away strength abruptly declines when the length of the head plug reaches 0.150mm with the safety factor above the standardized level (break-away strength: 50gf) taken into consideration.

Advantages in accompany with the reduced head plug 41 are described in detail hereinafter.

One of the reasons why the break-away strength of the head plug 41 is ameliorated is that the welding member 4 gives a certain amount of heat to the core wire 2 so as to increase its mechanical strength property in the first embodiment of the invention.

The core wire 2 used in the first embodiment of the invention is made from an austenitic stainless steel wire treated with a solid solution procedure.

With the use of an array of working dices, the austenitic stainless steel wire is drawn in a wire-drawing procedure until the wire diameter of the stainless steel wire comes from 1.00mm-2.28mm to 0.228mm-0.340mm. The wire-drawing procedure (work-hardening procedure) and the low-heat treatment (450°C for 30 minutes) are alternately repeated several times to increase the tensile rupture strength.

With use of the centerless grinder or the like, the distal end portion 21 of the core wire 2 is ground so that its wire diameter comes to 0.200mm-0.060mm with the distal end tip tapered off. Thereafter, a polishing procedure may be provided to smooth the outer surface of the core wire 2 by means of an electrolytic polishing, an emery paper or the like.

The reason why the polishing procedure is provided, is to remove an oxidized surface from the core wire 2 so as to improve the welding property of the welding member 4 since the core wire 2, which is highly drawn with its whole cross sectional reduction ratio as 80% or more, exceedingly deteriorates the wetting property with welding member 4.

By polishing the distal end portion 21 of the core wire 2 along its lengthwise direction, it is possible to equalize the machining injury to which the wire core 2 is subjected in the latitudinal direction due to the centerless grinder, thus protecting the core wire 2 against the breakage due to the machining injury, so as to improve the fatigue-resistant property against the repetitive bending action.

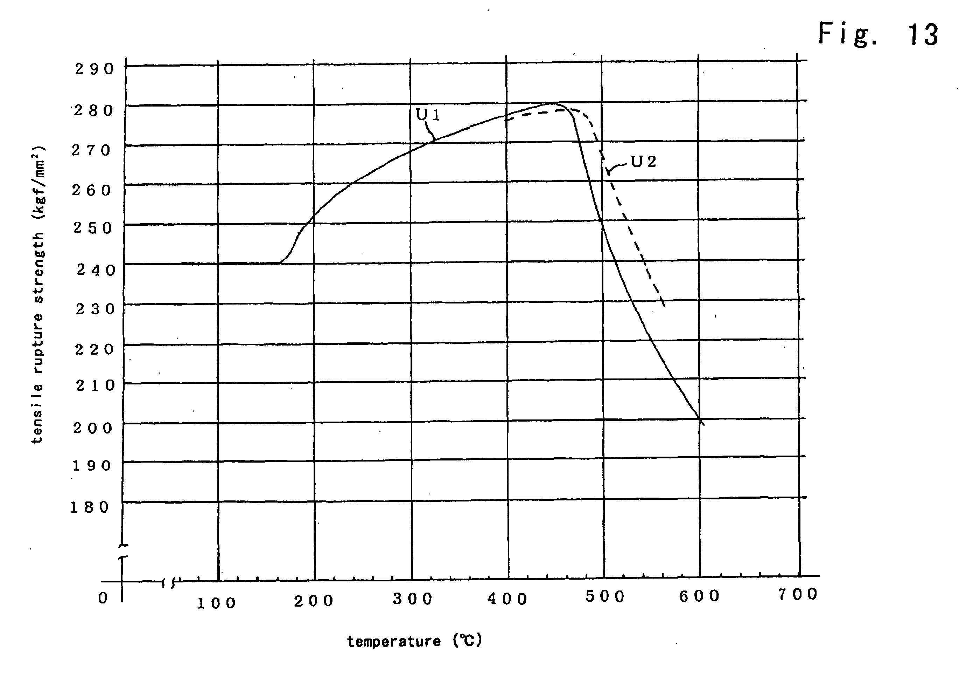

A graphical representation shown at solid line U1 in Fig. 13 is how the core wire 2 changes its tensile rupture strength property depending on the heating temperature of the core wire 2 (heated for 25 minutes).

As a specimen, taken is the core wire 2 (1.5mm in diameter) which is made of the austenitic stainless steel (SUS304) treated as the solid solution, and drawn until the whole cross sectional reduction ratio comes to 94.5% with wire diameter rendered as 0.350mm. The outer surface of the core wire 2 is ground so that its wire diameter comes to 0.100mm.

By heating the core wire 2 from 20°C (normal temperature) to 180°C, the temperature rise brings the tensile rupture strength from 240kgf/mm² to 248kgf/mm2 which means approximately 3.3% rise of the tensile rupture strength.

When heated to 280°C , the tensile rupture strength comes to 267kgf/mm², which means approximately 11.3% rise of the tensile rupture strength. When heated to 450°C , the tensile rupture strength is maximized to 280kgf/mm² which means approximately 16.7% rise of the tensile rupture strength.

When further heated to 495°C , the tensile rupture strength increases to 250kgf/mm² which means approximately 4.2% rise of the tensile rupture strength.

When the distal end portion 21 of the core wire 2 has 0.060mm in diameter, its tensile rupture strength increases from 678gf to 791gf upon rising the temperature from 20°C to 180°C. This means that the tensile rupture strength increases by approximately 113gf.

When heated to exceed 500°C , the tensile rupture strength abruptly declines due to the susceptive phenomenon of the stainless steel wire, and the tensile rupture strength comes to 200kgf/mm² upon heating to 600°C . This means that the tensile rupture strength substantially falls from approximately 791gf to approximately 565gf. When heated to exceed approximately 800°C , the core wire 2 permits the distal end portion 21 to rupture with a limited increase of tensile force. The magnitude of tensile force is so limited that it becomes difficult to design the core wire 2 even with a safety factor taken into consideration.

By using a eutectic alloy to the welding member 4 considering how the thermal influence extends to the tensile rupture strength of the core wire 2, it becomes possible to increase the tensile rupture strength property of the core wire 2 upon heating the core wire 2 at the time of forming the head plug 42 or the film layer 44 by means of the welding member 4.

Unless the eutectic alloy (welding member 4) is used with the above points taken into consideration, the eutectic alloy deteriorates the tensile rupture strength due to the melting heat produced at the time of welding the helical spring body 3 to the core wire 2 by means of the welding member 4 albeit the core wire 2 is work-hardened by means of the drawing procedure to increase the tensile rupture strength.

Especially as observed at the solid line U1 in Fig. 13, the tensile rupture strength sharply increases from 180°C to 220 °C, gradually ascending from 280°C to 300°C culminating at 450°C and still improved until 495°C. From 520°C and beyond, the tensile rupture strength abruptly falls more than the strength exhibited at 20°C (normal temperature).

Represented at dotted lines U2 in Fig. 13 is an austenitic stainless steel wire (SUS316) which contains 2%-3% molybdenum (Mo) to form the same type of the austenitic stainless steel wire (SUS304). As for the tensile rupture strength, the austenitic stainless steel wire (SUS316) exhibits the same propensity as the austenitic stainless steel wire (SUS304) does in the relatively low temperature range.
The austenitic stainless steel wire (SUS316) exhibits the maximum tensile rupture strength in the proximity of 480°C and continuously improves the tensile rupture strength until 525°C. From 540°C and beyond, the tensile rupture strength abruptly falls more than the strength exhibited at 20°C (normal temperature).

In order to improve the tensile rupture strength of the core wire for the austenitic stainless steel wire (SUS304), it is necessary to heat the core wire at 180°C-495°C, preferably 220°C -495°C and more preferably 280°C-495°C.

As for the austenitic stainless steel wire (SUS316), it is necessary to heat the core wire at 180°C-525°C , preferably 220°C -525°C and more preferably 280°C-525°C.

When the core wire is drawn so that its wire diameter reduces from 1.500mm to 0.340mm, the whole cross sectional reduction ratio comes to 94.8%. When the core wire is drawn so that its wire diameter reduces from 1.500mm to 0.228mm, the whole cross sectional reduction ratio comes to 97.6% with the tensile rupture strength determined to be 300kgf/mm².

By drawing the austenitic stainless steel wire treated as the solid solution (2.28mm in diameter with the tensile rupture strength as 70kgfmm²-80kgf/mm²) until the wire diameter comes to 0.228mm, the whole cross sectional reduction ratio comes to 99.0% to exhibit a high tensile rupture strength which extends beyond 350kgf/mm² to reach near 400kgf/mm².

It is preferable to determine the whole cross sectional reduction ratio to be 80% or more, otherwise 90% or less, more preferably 90% or more, otherwise 99% or less.

In this instance, the whole cross sectional reduction ratio R means a reduction rate expressed by R=(S1-S2)/S1.

Where S1 is a cross sectional area regarding the original diameter of the solid solution wire before the wire is drawn, and S2 is a resultant cross sectional area regarding the finished diameter of the solid solution wire after the wire is drawn.

The whole cross sectional reduction ratio is preferably determined to be 80% or more because the tensile rupture strength changes at the ratio R of 80%, and abruptly increases when the ratio R extends beyond 80% as a point of inflection. As for the stainless steel wire used for a helical spring, the whole cross sectional reduction ratio is determined to be 80%-90% as described on page 62 (Figure Number 2 · 82) of "Manual on Spring, Third Edition" published by "Maruzen Incorporation".

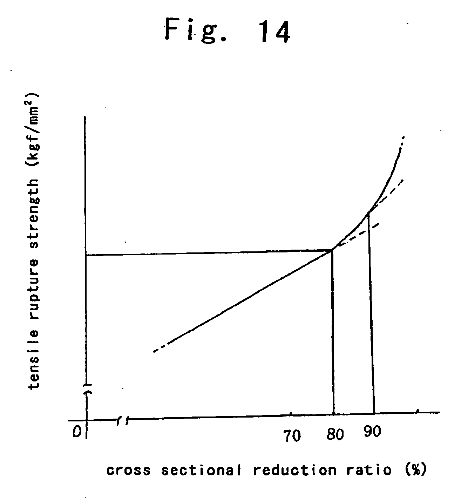

The whole cross sectional reduction ratio is more preferably determined to be 90.0% or more because the tensile rupture strength sharply increases when the whole cross sectional reduction ratio comes to 90% and extends beyond 90% as shown in Fig. 14.

This is because the austenitic stainless steel wire is plastically wrought out tightly during the drawing procedure, so that the stainless steel wire develops a fibroid structure excessively when the whole cross sectional reduction ratio comes to 80% or more, especially 90% or more.

The whole cross sectional reduction ratio is determined to be 99% or less because the stainless steel wire develops minute voids within its structure to make the structure brittle when the whole cross sectional reduction ratio exceeds 99% as an upper drawing limit with the productivity taken into consideration.

That the austenitic stainless steel wire is drawn as the solid solution, is to provide the wire with superior workability.

Since it is hard to obtain the minute crystalloid of the austenitic stainless steel wire by making use of the transmutational point during the heat treatment process, instead of the heat treatment, the cold working process is used in order to achieve the minute crystalloid of the austenitic stainless steel wire, and the wire is work hardened to improve the tensile strength during the drawing process.

Another reason to use the austenitic stainless steel wire is that the martensitic stainless steel wire tends to be hardened during the quenching process and susceptible to the thermal influence, and a precipitation-hardened stainless steel wire lacks the toughness to be likely broken so as to render it difficult to form a flat section 23A on the core wire 2 in Fig. 8. The ferro-based stainless steel wire tends to be hot-short (sigma brittle, brittle at 475°C).

The reason why the break-away strength of the head plug 41 is improved in the first embodiment of the invention, is to use the welding member 4 with the tensile rupture strength of the core wire 2 taken into consideration.

In the first embodiment of the invention, the welding member 4 is made of the eutectic alloy which has the melting temperature in the range of 180°C-495°C.

The core wire 2 exhibits a tendency to increase the tensile rupture strength at 180°C as observed in Fig. 13, and ascending the tensile rupture strength rapidly at around 220°C through 280°C -300°C , and culminating the tensile rupture strength at 450°C , while gradually decreasing the tensile rupture strength at the temperature from 450°C to 495°C .

This makes it possible to weld the head plug to the core wire 2, the tensile rupture strength of which increases at the temperature of 180°C-495°C.

As for the core wire 2 made of the austenitic stainless steel which contains molybdenum (Mo), employed to the welding member 4 is the eutectic alloy which has the melting temperature in the range from 180°C-525°C.

This makes it possible to weld the head plug to the core wire 2, the tensile rupture strength of which increases at the temperature of 180°C-525°C.

By making good use of the melting heat derived from the welding member 4, it becomes possible to weld the head plug 41 to the core wire 2, while at the same time, increasing the tensile rupture strength of the core wire 2.

The eutectic alloy means a special alloyed metal, components of which can be adjusted to gain a lowest melting temperature.

As a gold-tin based alloy, it contains 80% gold by weight and 20% tin by weight to have the melting temperature of 280°C . As a silver-tin based alloy, it contains 3.5% silver by weight and 96.5% tin by weight to have the melting temperature of 221°C. As a gold-germanium based alloy, it contains 88% gold by weight and 12% germanium by weight to have the melting temperature of 356°C. As gold-tin-indium based alloys, they are represented to have the melting temperature of 450°C -472°C as shown in Table 1.

**Table 1**

| No. | Eutectic Alloy (%) by weight | Melting Temp |
|---|---|---|
| A-1 | gold (80%) tin (20%) | 280°C |
| A-2 | gold (10%) tin (90%) | 217°C |
| A-3 | gold (88%) germanium (12%) | 356°C |
| A-4 | gold (73.3%) indium (26.7%) | 451°C |
| A-5 | gold (94.0%) silicon (6.0%) | 370°C |
| B-1 | silver (3.5%) tin (96.5%) | 221°C |
| B-2 | silver (40%) tin (30%) indium (30%) | 450°C |
| B-3 | silver (40%) tin (40%) indium (10%) copper (10%) | 458°C |
| B-4 | silver (45%) tin (45%) indium (10%) | 472°C |
| B-5 | silver (5%) tin (95%) | 250°C |

The reason that the gold is used for the welding member 4, is to improve a visual recognition under the fluoroscopy, corrosion-resistance and ductility. The silver is used to adjust the melting temperature of the welding member 4, and the tin is to lower the melting temperature of the welding member 4 to increase the wetting property with the core wire 2 or the helical spring body 3.

This is true with the indium and copper. The germanium is used to suppress the intermetallic crystalline from turning coarse, so as to prevent the welding strength from reducing to an unacceptable degree.

It is to be noted that the use of antimony (stibium) is not suitable because of its non-biocompatibility and machining difficulty.

Reasons why the melting temperature of the welding member 4 in the range 180°C-495°C or 180°C-525°C are that it becomes difficult to increase the tensile rupture strength of the work-hardened core wire 2 by using the melting heat of the welding member 4 when the melting temperature decreases to less than 180°C. When the melting temperature exceed 495°C (525°C for the Mo-based austenitic stainless steel wire), the austenitic stainless steel wire decreases its tensile rupture strength significantly since when the austenitic stainless steel wire is heated to the temperature of 800°C which exceeds 520°C and 540°C, it becomes to require an energy to precipitate the carbon particles and mobilize chromium within the austenitic stainless steel wire (susceptive phenomenon), so as to exceedingly reduce the tensile rupture strength.

This makes to possible to impart the core wire 2 with a maximum mechanical strength by suppressing the susceptive phenomenon appeared on the austenitic stainless steel wire.

Upon using the silver-based brazing having the melting temperature of 605°C-800°C or the gold-based brazing having the melting temperature of 895°C-1030°C as the welding member 4, the melting heat significantly decreases the tensile rupture strength of the core wire 2 because the core wire 2 is annealed or becomes brittle due to the susceptive phenomenon. This increases the possibility that the head plug 41 comes off the core wire 2 or the helical spring body 3.

Reasons why the head plug 41 increases its break-away strength are that the welding heat of the welding member 4 subjects the partial heat treatment to a distal region (especially observed at the region N0 in Fig. 5) of the core wire 2 to which the head plug 41 is welded, and the partial heat treatment increases the wetting property between the core wire 2 and the welding member 4, so as to improve the welding strength and the tensile rupture strength therebetween.

The welding heat of the welding member 4 extends the heat treatment to the distal end portion 21 of the core wire 2 which lies at a distal end region in the rear of the head plug 41 , so as to improve the fatigue-resistant property against the repetitive bending action to which the distal end portion 21 of the core wire 2 is subjected.

This is due to the reason that the heat treatment increases the tensile rupture strength of the core wire 2 to decrease the residual angle which the core wire 2 forms upon returning to the original shape after bent to a certain degree.

When the core wire 2 is the austenitic stainless steel wire with the whole cross sectional reduction ratio as 90% or more, the core wire 2 is heat treated from the distal end tip to the proximal extension (1.0mm-30.0mm in length) of the core wire 2 at 220°C-495°C for 1/60-60 minutes, preferably 280 °C-495°C for 1/60-60 minutes.

The heat treatment may be carried out by the hot air atmosphere with the use of a heat treating furnace, or through the thermal heat conduction of the soldering iron, or by heating pin-point portion (1.0mm-2.0mm in width) of the head plug 41 at the welded portion in the nitrogen atmosphere.

Table 2 shows experimentation test results of the specimens A, B, each taken thirty as test lot number.

After passing the specimens A, B through a U-shaped pipe (2.0mm in inner diameter), the specimens A, B are looked carefully how far the specimens A, B are angularly deformed as a residual angle (θ) from the initial straight line against the dog-legged line represented after released from the U-shaped pipe.

The specimen A is the core wire 2 made of the austenitic stainless steel wire (0.06mm in diameter), and partially heat treated at 450°C for 2 minutes at the certain extension (20mm in length) from the distal end tip of the core wire as prepared in the first embodiment of the invention.

The specimen B is a comparative core wire which is not heat treated at all.

**Table 2**

| residual angle | specimen A | specimen B |
|---|---|---|
| angle (θ) | 15-20 deg. | 42-50 deg. |
| average angle | 17.5 deg. | 46 deg. |

Table 2 shows that the specimen A exhibits the residual angle (θ) less than half the angle which the specimen B does, so as to show that the specimen A remains a small amount of deformation after passing through the U-shaped pipe. The specimen A exhibits a factor 795 as Vicker's hardness (HV), which is higher by approximately 45 than that of the specimen B with the tensile rupture strength increased by approximately 6% from 263kgf/mm² to 279kgf/mm².

From the experimentation test results in Table 2, it can be observed that the tensile rupture strength is improved by partially heat treating the highly drawn core wire, while at the same time, ameliorating the pushability and fatigue-resistant property with a small amount of residual angle (θ).

Even if the specimen A is heated at 450°C only for 1 second, the specimen A increases the tensile rupture strength by increasing the Vicker's hardness (HV) by the factor of approximately 10. This is because the core wire 2 is as thin as 0.06mm in diameter, so that the distal end portion 21 is very vulnerable to the thermal influence with a small heat capacity.

It is to be appreciated that the lower limit of the heating duration for the core wire 2 is preferably 3 seconds or more, more preferably 10 seconds or more within the prescribed heating temperature and time duration.

Reasons why the break-away strength is increased is that the film layer 44 is coated with the distal end portion 21 of the core wire 2, and the minor weld-hardened portion 412 is formed with the use of the welding member 4 which has the same or same type of the eutectic alloy of the film layer 44.

Further, the head-most portion 411 is formed in the rear of the minor weld-hardened portion 412 with the use of the welding member 4, so as to form the head plug 4 with the head-most portion 411 and the minor weld-hardened portion 412 through the film layer 44.

Even without the film layer 44, it is possible to increase the break-away strength so long as the head plug 41 is formed from the head-most portion 411 and the minor weld-hardened portion 412 with the use of the welding member 4 which has the same or same type of the eutectic alloy of the head plug 41.

The eutectic alloy the same type of the welding member 4 means that the gold, silver or tin, otherwise two of them occupy 50% by weight or more among a total components of the eutectic alloy. In Table 1, the eutectic alloys designated by A1 - A4 and B1 - B4 are the same type, but those designated by the combination of A1 - A4 and B1 - B4 belong to different types of eutectic alloys.

Reason why the film layer 44 is coated with the distal end 21 of the core wire 2, is that the film layer 44 reduces the contact angle to improve the wetting property with the head-most portion 411 and the minor weld-hardened portion 412 so as increase the welding strength with the core wire 2.

Reason why the minor weld-hardened portion 412 is formed from the same or same type of the eutectic alloy as the film layer 44 (welding member 4), is to improve the wetting property between the core wire 2 and the head plug 41 with the welding strength ameliorated therebetween. This is true when the head-most portion 411 is formed from the same or same type of the eutectic alloy as the minor weld-hardened portion 412 (welding member 4).

Although it is preferable that the length of the region N0 is 1.0mm-8.0mm, the length N1 in Fig. 5 is preferably 2.0mm or less from the rear end side of the head plug 4 including the core wire 2 in which the head plug 4 is placed. The length N1 is more preferably 0.5mm-1.0mm, and most preferably 0.0mm, the latter of which means to place the film layer 44 only within the length of the head plug 41.

In order to reduce the length of the head plug 41 , the head plug 41 is formed from the minor weld-hardened portion 412 by severing the weld-hardened portion 413 together with core wire 2 and the helical spring body 3. Advantages obtained by reducing the length of the head plug 41 are described in detail hereinafter.

Reason why the head plug increases the break-away strength is due to the minor weld-hardened portion 412 invaded into the clearance P between the helices of the helical spring body 3, and securing an increased contact area between the head plug 41 and the distal end portion 21 of the core wire 2 (anchor effect). Especially, the anchor effect is obtained when the distal end portion 21 of the core wire 2 is flatten as shown in Fig. 8, 9.

Namely, the helical spring body 3 has the clearance P (Fig. 5), the width of which is 5%-85% of the wire diameter of the helix of the helical spring body 3, and the outer diameter D3 of the minor weld-hardened portion 412 is greater than a central diameter D0 of the helical spring body 3 but smaller than an outer diameter D2 of the helical spring body 3.

A part of the helices of the helical spring body 3 is embedded in the minor weld-hardened portion 412. It is to be noted that the central diameter D0 means an average diameter ((D1 + D2)/2) between the outer diameter D2 and inner diameter D1 of the helical spring body 3.

Embedded in the minor weld-hardened portion 412 is the helices of the helical spring body 3 so that the contact area against the minor weld-hardened portion 412 increases with a limited portion of the helical spring body 3, so as to increase the break-away strength of the head plug 41 due to the anchor effect.

By forming the flat section 23A on the core wire 2 (Fig. 8), or providing an array of minute grooves 5a at one side or both sides of the flat surface 5 (Fig. 9), it becomes possible to increase the contact area against the minor weld-hardened portion 412 so as to improve the break-away strength against the core wire 2.

More specifically, by flattening the distal end portion 21 of the core wire 2 (0.06mm in diameter) to form the flat section 23A rectangular in cross section, the flat section 23A measures 0.094mm in width and 0.030mm in thickness. This makes it possible to increase the contact area by 1.32 fold compared to the core wire circular in cross section.

By providing the array of grooves 5a which measures 0.003mm-0.005mm in depth, it is possible to reinforce the anchor effect. The array of grooves 5a is preferably in perpendicular to the lengthwise direction of the core wire 2.
Instead of the array of grooves 5a, a latticework pattern may be provided on the flat section 23A of the core wire 2.

With the increased cross sectional area of the flat section 23A, the array of grooves 5a synergistically work the anchor effect to firmly retain the film layer 44 to the flat section 23A.

By producing the guide wire 1 in which the length of the head plug 41 is reduced to increase the break-away strength, it becomes possible to significantly increase the chance of success upon therapeutically treating a completely occluded lesion (chronic disease) of the cardiovascular system.

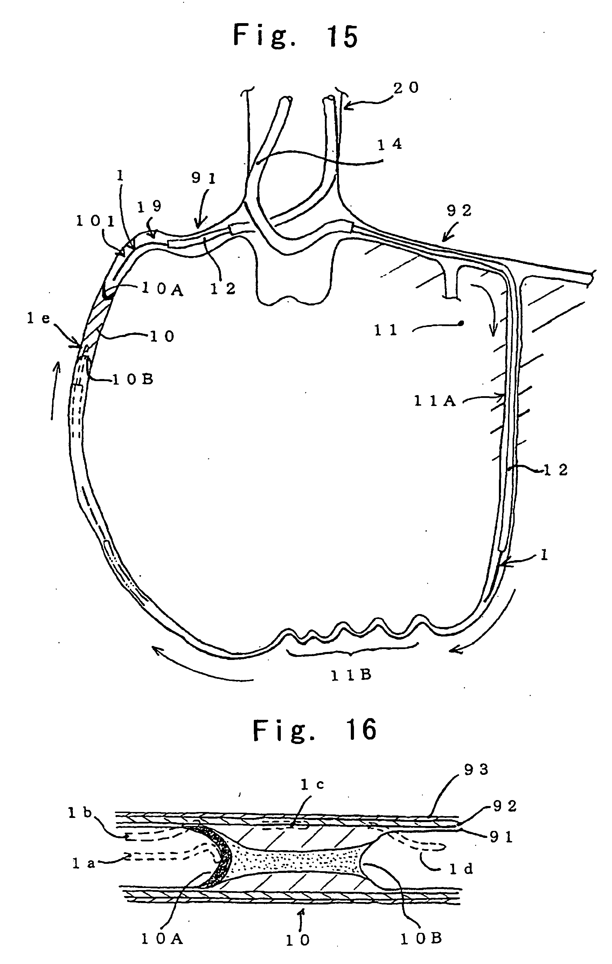

Fig. 16 shows an example of clinically treating the completely occluded lesion 10 as disclosed by Japanese Laid-open Patent Application No. 2003-164530 (first reference). In the completely occluded lesion 10 of the coronary artery, a front occlusion end 10A located on the proximal side of the aorta has a fibrous cap harder than that of a rear occlusion end 10B located on the distal side of the aorta. Upon inserting the guide wire 1 into the coronary artery to encounter the front occlusion end 10A, the guide wire 1 makes its distal end 1 a curvedly deform, thereby making it difficult to perforate the front occlusion end 10A.

In order to avoid the encounter against the front occlusion end 10A, the guide wire 1 is adjusted to move by 2mm-3mm in a rear and front direction by a push-pull operation, and introduced from an entrance 1b, through a boundary 1c between the intima 91 and the media 92 to the other side beyond the occluded lesion 10 by feeling the distinction between the rough touch of the intima 91 and the sticky touch of the media 92 inside the adventitial coat 93 within the coronary artery.

Such is the therapeutical treatment that the manipulation requires a highly trained skill and an extended time period for the operator to acquire.

In recent years, however, it has been found that the rear occlusion end 10B is soft to the touch compared to the front occlusion end 10A which directly admits the blood streams from the aorta.

The guide wire 1 has been manipulatively inserted from the rear occlusion end 10B toward the front occlusion end 10A (referred to as "reverse approach technique") so as to perforate the rear occlusion end 10B successfully.

Upon carrying out the reverse approach technique, it is important to find the collateral path, i.e., the blood vessel which nutritionally eats the periphery of the occluded lesion 10 in the ceptal 11 as observed in Fig. 15.

The ceptal collateral path 11A is the blood vessel developed as a self-defensive function due to the appearance of the occluded lesion 10, contrary to the blood vessel before the occluded lesion 10 develops.

For this reason, the ceptal collateral 11A meanders to form a highly sinuous cork screw vessel 11B which has 6-8 U-shaped paths at the length of approximately 50mm with the radius of curvature calculated as approximately 3.0mm.

About 50%-60% of the ceptal collateral path 11A has an outer diameter less than approximately 0.4mm, contrary to the outer diameter (3.0mm-4.0mm) of the coronary artery.

Upon navigating the guide wire 1 through the ceptal collateral path 11A, it is necessary to determine the distal end portion of the guide wire 1 to be 0.4 mm or less in diameter.

Further, it becomes necessary for the head plug 41 to have a reduced length and a capability of making a small turn in order to follow the meandering path upon navigating the guide wire 1 through the sinuous cork screw vessel 11B.

For this reason, the head plug 41 has the length L of approximately 0.190mm-0.60mm as the most preferable mode, contrary to the length (approximately 1.0mm) of the counterpart head plug in Japanese Laid-open Patent Application No. 2005-6868 (fourth reference).

In order to navigate the guide wire 1 through the ceptal collateral 11A, it is necessary to determine the outer diameter D4 of the head plug 41 to be preferably 0.345mm or less, more preferably 0.305mm or less, most preferably 0.254mm or less.

This is because about 50%-60% of the ceptal collateral path 11A has an outer diameter less than approximately 0.4mm, and the guide wire 1 is required to have the capability of making a small turn in order to follow the meandering path upon navigating the guide wire 1 through the sinuous cork screw vessel 11B.

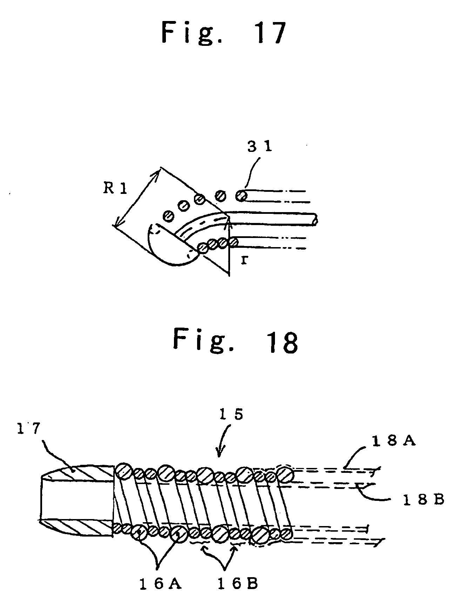

In order for the guide wire 1 to make a small turn, it is necessary for the guide wire 1 to have a bending propensity at the time of preshaping it, and having a sufficiently reduced length R1 in the axial direction with a small radius of curvature (r) as shown in Fig. 17.

The head plug 41 is well suited upon navigating the guide wire 1 through the sinuous cork screw vessel 1 1 B with the increased break-away strength maintained.

It is to be noted that the preshaped configuration is imparted to the distal end portion of the guide wire 1 together with the radiopaque coil 31 by bending the distal end portion plastically with the bending force exceeding the elastic deformation.

As for the length N1 (Fig. 5), since the smaller the length N1 becomes, the more flexible the core wire 2 becomes to have the capability of making a small turn, the length N1 is preferably 0.5mm-1.0mm, most preferably 0.0mm as described hereinbefore.

Upon passing the guide wire 1 through the sinuous cork screw vessel 11B in a second embodiment of the invention, it is necessary to prepare an assembly of a microcatheter 12 and a guiding catheter 14 combined with the guide wire 1 in order to support a reactional force which advances the guide wire 1 through the coronary artery as shown in Fig. 15.

Upon implementing the therapeutical treatment against the completely occluded lesion 10, the guide wire 1 is inserted into a microcatheter 12 (0.28mm-0.90mm in inner diameter), and the guide wire 1 inserted into the microcatheter 12 is further inserted into a guiding catheter 14 (1.59mm-2.00mmin inner diameter) together with the microcatheter 12.

Such is the structure that it enables the operator to advance the guide wire 1 through the cork screw vessel 11B, and encounter the rear occlusion end 10B to readily perforate the completely occluded lesion 10 as shown at dotted lines (1e) in Fig. 15.

In Fig. 15, numeral 19 designates a therapeutical procedure to introduce the guide wire 1 against the completely occluded lesion 10 from the front occlusion end 10A (forward approach technique). Numeral 91 designates the right coronary artery, numeral 92 the left coronary artery, and numeral 20 the aortic arch.

It is preferable to use the gold metal as the eutectic alloy for the welding member 4 in order to prevent the welding strength from decreasing due to the corrosion, avoiding the head plug 42 from darkening, and preventing the visual recognition of the head plug 41 from fading away under the fluoroscopy.

This is because the guide wire 1 is usually dipped in the physiological saline solution before using the guide wire 1, and silver sulfide appears on the head plug 41 to darken the head plug 41 within one hour after dipping the guide wire 1 when the silver-based eutectic alloy is used to the head plug 41.

With the passage of time, the silver sulfide deeply darkens the head plug 5 to decrease the welding strength due to the corrosion. Additionally, since the guide wire 1 is diametrically thinned, it is necessary to avoid the visual recognition of the guide wire 1 from fading away when passing through the sinuous cork screw vessel 11B.

The austenitic stainless steel wire of the present invention has chemical composition as follows:
C: less than 0.15% by weight, Si: less than 1.0% by weight, Mn: less than 2.0% by weight, Ni: 6%-16% by weight,

Cr: 16%-20% by weight, P: less than 0.045%, S: less than 0.030%, Mo: less than 3.0%, balance: iron and impure substances unavoidably contained.

Without using a high silicic stainless steel (Si: 3. 0%-5.0% by weight) or the precipitation-hardened stainless steel wire, it is possible to provide the core wire 2 with a high tensile strength by means of the austenitic stainless steel wire. This is achieved by repeatedly applying one or three sets to the austenitic stainless steel wire with the drawing procedure and the low heat treatment (450°C for 30 minutes) combined as a single one set before finally drawing the austenitic stainless steel wire.

It is preferable to add 0.005% as a carbon component to increase the tensile rupture strength, and add 0.15% as the carbon component to prevent the intergrannular corrosion.

It is preferable to use the austenitic stainless steel wire (redissolved SUS304 or SUS316) to draw the core wire 2 (0.228mm-0.355) with the tensile rupture strength as more than 300kgf/mm² and the whole cross sectional reduction ratio as more than 95%.

Most of the causes that the stainless steel wire is disconnected upon drawing the stainless steel wire, is due to the oxidized substances rather than scars incurred on the outer surface of the stainless steel wire. This likelihood becomes remarkable as the stainless steel wire is deeply drawn with the increase of the whole cross sectional reduction ratio.

The oxidized substances cause cracks and injuries to occur on the stainless steel wire especially upon pressing the distal end portion 21 of the core wire 2 (0.060mm in diameter) to be rectangular in cross section which measures 0.094mm in width and 0.030mm in thickness.

It is preferable to reduce the chemical components Si, Al, Ti and O which are elements of the oxidized substances. This is true with the sulfur which causes to reduce the drawing capability of the stainless steel wire. It is preferable to add an appropriate amount of copper which improves the drawing capability when the copper is added to the stainless steel wire by 0.1% or more by weight.

With the above matters in mind, the austenitic stainless steel wire needs the chemical composition as follows:
C: less than 0.08% by weight, Si: less than 0.10% by weight, Mn: less than 2.0% by weight, P: less than 0.045% by weight, S: less than 0.10% by weight, Ni: 8%-12% by weight, Cr: 16%-20% by weight, Mo: less than 3.0% by weight, Cu: 0.1%-2.0% by weight, Al : less than 0.0020% by weight, Ti: less than 0.10% by weight, Ca: less than 0.0050% by weight, O : less than 0.0020% by weight, and balance: iron and impure substances unavoidably contained.

Upon manufacturing redissolved materials, the flux is used to an ingot of the resolved stainless steel as the electro-slug redissolving method. The triple dissolving material may be used.

A method of making the guide wire 1 according to the present invention are as follows:

The core wire 2 is formed by a flexible elongate member with the helical spring body 3 inserted to a distal end portion 21 of the core wire 2 to be placed around the core wire 2. The head plug 41 is provided at distal end tips of both the core wire 2 and the helical spring body 3 by means of a welding member 4. Drawn is the core wire 2 which is made of an austenitic stainless steel wire treated with a solid solution procedure until a whole cross sectional reduction ratio R of the core wire 2 reaches 90%-99%. The distal end portion 21 of the core wire 2 is ground. The helical spring body 3 is assembled by inserting the helical spring body 3 to the distal end portion 21 of the core wire 2 to be placed around the core wire 2. The weld-hardened portion 413 is formed at the welded portion between the distal end tips of the core wire 2 and the helical spring body 3 by melting the welding member 4 as a eutectic alloy having a melting temperature of 180°C-495°C including a eutectic alloy having a melting temperature of 180°C-525°C when the austenitic stainless steel wire of the core wire 2 has molybdenum (Mo). The minor weld-hardened portion 412 is formed by severing the weld-hardened portion 413 at the predetermined length H from a distal tip portion of the weld-hardened portion 413. The head-most portion 411 is provided by the same or same type of the welding member 4 from which the minor weld-hardened portion 412 is made, and attaching the head-most portion 411 in integral with the distal tip portion of the minor weld-hardened portion 412 so as to form the head plug 41.

With the method as mentioned above, it is possible to make good use of the welding heat of the welding member 4 so as increase the tensile rupture strength of the core wire 2, and synergistically improving the welding strength of the core wire 2 at the welded portion (head plug 41) between the core wire 2 and the helical spring body 3 with the austenitic stainless steel wire deeply drawn as the core wire 2, thereby making it possible to lengthwisely reduce and diametrically minimize the head plug 41 with a good maneuverability imparted to the core wire 2.

It is to be noted that the distal end portion 21 of the core wire 2 may be polished between the procedure of grinding the core wire 2 and the procedure of inserting the helical spring body 3 to the core wire 2.

After grinding the distal end portion 21 of the core wire 2, at least at a portion of the distal end portion 21 in which the head plug 41 is formed, is partially heat treated at 180°C-495°C for 1/60-60 minutes. For the core wire 2 in which the austenitic stainless steel wire has the molybdenum (Mo) as its component element, the above portion is partially heat treated at 180°C-525°C for 1/60-60 minutes.

This makes it possible to increase the tensile rupture strength of the core wire 2 highly drawn due to the tightly drawing procedure, while at the same time, increasing the welding strength between the core wire 2 and the welding member 4 by improving the wetting property therebetween.

Further, during implementing the partial heat treating procedure for 1/60-60 minutes, the film layer 44 is formed on the distal end portion 21 of the core wire 2 with the use of the welding member which is the same or same type of the welding member from which the head plug 41 is made.

The welding member increases the wetting property between the core wire 2 and the head plug 41, while at the same time, improving the welding strength between the head-most portion 411 and the minor weld-hardened portion 412 which are integrally attached together to form the head plug 41. This leads to lengthwisely reducing and diametrically minimizing the head plug 41.

With the welding strength of the core wire 2 increased against the head plug 41, it becomes possible to diametrically thin the core wire 2 in which the tensile rupture strength is already increased by applying the austenitic stainless steel wire to the core wire 2.

By way of illustration, it is possible to dimensionally reduce outer diameters of the proximal portion 22 of the guide wire 1 and the helical spring body 3 from 0.355mm (0.014 inches) to 0.254mm (0.010 inches) when the core wire 2 is made of austenitic stainless steel wire, and further it becomes possible to dimensionally reduce outer diameters (D4, D2) of the head plug 41 and the helical spring body 3 from 0.355mm (0.014 inches) to 0.228mm (0.009 inches) by increasing the welding strength between the head plug 41 and the helical spring body 3.

Upon forming the assembly of the microcatheter 12 and the guiding catheter 14 combined with the guide wire 1 , the guide wire 1 is inserted into a microcatheter 12, and the guide wire 1 inserted into the microcatheter 12 is further inserted into a guiding catheter 14 together with the microcatheter 12.

In accompany with the guide wire 1 being thinned, the guiding catheter 14 is also thinned from 7F-8F (2.3mm-2.7mm in inner diameter) to 5F-6F (1.59mm-2.00mm in inner diameter), while at the same time, thinning the balloon catheter 13 to be 0.28mm-0.90mm in inner diameter.

This makes it possible to render the guide wire 1 minimally invasive so as to mitigate the burden from which the patient suffers when therapeutically treated.

With the thinned guide wire 1 as mentioned above, it becomes possible to readily implement the "reverse approach technique" to pass through the ceptal collateral path 11A, so as to increase a chance of success to a significant degree upon treating the completely occluded lesion 10 as the chronic disease.

In addition, upon inserting the microcatheter 12 and the guide wire 1 to the entry of the occluded lesion 10 through the coronary artery as shown in Fig. 15, the microcatheter 12 supports the reactional force against the pushing force to which the guide wire 1 is subjected, so as to favorably advance the guide wire 1 through the coronary artery.

As a third embodiment of the invention, the microcatheter 12 is made of multi-layered synthetic tubes with inner and outer layers made of polytetrafluoroethylene (PTFE) and polyamide respectively, or multi-layered synthetic tubes which are strengthened by braided thin wires.

Otherwise, as a helical tube body, the microcatheter 12 is a wire-wound tube body 15 made by winding a plurality of wires in a spiral fashion with a cone-shaped metal tip 17 provided on a distal end thereof as shown in Fig. 15.

This makes the wire-wound tube body perforative against an obstructed area within the completely occluded lesion 10.

Upon passing the microcatheter 12 through the ceptal collateral path 11A which minutely meanders with a samll diameter, it is preferable to use the wire-wound tube body 15, an outer surface of which undulates in a concave-convex fashion along the lengthwise direction.

The wire-wound tube body 15 may be made by alternately winding or stranding thick wires 16A (1-2 wires with a diameter as 0.11mm-0.18mm) and thin wires 16B (2-8 wires with a diameter as 0.06mm-0.10mm).

The outer surface of the wire-wound tube body 15 undulates in a concave-convex fashion because the wire-wound tube body 15 tightly contacts the outer surface with the vascular wall of the coronary artery, thereby preventing the slip against the vascular wall so as to tightly support the guide wire 1 when subjected to the reactionary force of the guide wire 1.

The wire-wound tube body 15 makes the thick wires 16A firstly contact with the vascular wall and advance longer when manipulatively rotated by one turn, so as to quickly move the assembly in the reciprocal direction because the wire-wound tube body 15 moves back and forth along the vascular wall along a diametrical pitch of the thick wires 16A.

It is to be noted that synthetic layers 18A, 18B may be formed on inner and outer surfaces of the wire-wound tube body 15 respectively so long as the concave-convex is provided on a part or entirety of the wire-wound tube body 15. This holds true when the concave-convex is partly provided on the outer surface of the wire-wound tube body 15 by a predetermined length (e.g., 300mm from a distal end tip rearward) which is subjected to the pressing or pushing action from the vascular wall when inserted into the occluded lesion 10 of the coronary artery.

With the use of the head plug 41, it is possible to diametrically thin the guide wire 1. The guide wire 1 is inserted into the balloon catheter 13 which is inserted into the guiding catheter 14.

In association with the guide wire 1 being thinned, the guiding catheter 14 is also thinned from 7F-8F (2.3mm-2.7mm in inner diameter) to 5F-6F (1.59mm-2.00mm in inner diameter), while at the same time, thinning the balloon catheter 13 to be 0.28mm-0.90mm in inner diameter.

This makes it possible to render the guide wire 1 minimally invasive so as to mitigate the burden from which the patient suffers when therapeutically treated, and enabling the operator to readily implement the "reverse approach technique" so as to significantly increase a chance of success upon treating the completely occluded lesion 10 as the chronic disease.

As apparent from the foregoing description, according to the present invention, it is possible to increase the welding strength between the core wire 2 and the helical spring body 3, whereby lengthwisely reducing and diametrically minimizing the head plug 41 to diametrically thin the core wire 2 so as to increase a chance of success for therapeutically treating the completely occluded lesion 10 by means of the specific technique.

By observing the relationship between the temperature and the tensile rupture strength of the core wire 2, it becomes possible to increase the welding strength between the core wire 2 and the plug head 41, while at the same time, increasing the tensile rupture strength with the use of the melting heat produced upon melting the welding member 4 to form the plug head 41.

## Claims

1. A method of making a medical guide wire (1) having a core wire (2) formed by a flexible elongate member, a helical spring body (3) inserted to a distal end portion (21) of said core wire (2) to be placed around said core wire (2), and a head plug (41) provided at distal end tips of both said core wire (2) and said helical spring body (3) by means of a welding member (4), **characterised by** steps of:
drawing said core wire (2) which is made of an austenitic stainless steel wire treated with a solid solution procedure until a whole cross sectional reduction ratio of said core wire (2) reaches 90%-99%;
grinding said distal end portion (21) of said core wire (2);
assembling said helical spring body (3) by inserting said helical spring body (3) to said distal end portion (21) of said core wire (2) to be placed around said core wire (2) ;
forming a weld-hardened portion (413) at a welded portion between said distal end tips of said core wire (2) and said helical spring body (3) by melting a welding member (4) as a eutectic alloy having a melting temperature of 180°C -495°C including a eutectic alloy having a melting temperature of 180°C - 525°C when said austenitic stainless steel wire of said core wire (2) has molybdenum as a component ;
forming a minor weld-hardened portion (412) by severing said weld-hardened portion (413) at a predetermined length from a distal tip portion of said weld-hardened portion (413) ; and
providing a head-most portion (411) by the same or same type of a welding member (4) from which said minor weld-hardened portion (412) is made, and attaching said head-most portion (411) in integral with a distal tip portion of said minor weld-hardened portion (412) so as to form said head plug (41) ;
wherein said whole cross sectional reduction ratio means a reduction rate R expressed by R=(S1-S2)/S1, in which S1 is a cross sectional area regarding an original diameter of said core wire (2) treated with said solid solution procedure before said core wire (2) is drawn, and S2 is a resultant cross sectional area regarding a finished diameter of said core wire (2) after said core wire (2) is finally drawn during said drawing process.

2. The method of making said medical guide wire (1) according to claim 1, wherein after grinding said distal end portion (21) of said core wire (2), a distal end of said core wire (2) is heat treated partially under a temperature of 180°C-495°C at least at a portion in which said head plug (41) is formed, including a temperature of 180°C -525°C when said austenitic stainless steel wire of said core wire (2) has molybdenum as a component, and a welding member (4) is melted to be poured over an outer surface of said core wire (2) by a predetermined length thereof so as to form a film layer (44), so that said distal end of said core wire (2), said minor weld-hardened portion (412) and said head-most portion (411) are united to form said head plug (41), said welding member (4) being the same or same type of a welding member as used when said head plug (41) is formed.

## Patentansprüche

1. Verfahren zum Herstellen eines medizinischen Führungsdrahts (1) mit einem durch ein biegsames längliches Element gebildeten Kerndraht (2), einem Schraubenfederkörper (3), der zu einem distalen Endabschnitt (21) des Kerndrahts (2) eingeführt ist, um um den Kerndraht (2) positioniert zu werden, und einem Kopfstopfen (41), der an distalen Endspitzen sowohl des Kerndrahts (2) als auch des Schraubenfederkörpers (3) mittels eines Schweißelements (4) vorgesehen ist, **gekennzeichnet durch** folgende Schritte:
Ziehen des Kerndrahts (2), der aus einem mit einem Festlösungsvorgang behandelten Austenitedelstahldraht hergestellt ist, bis ein gesamtes Querschnittreduktionsverhältnis des Kerndrahts (2) 90% - 99% erreicht;
Schleifen des distalen Endabschnitts (21) des Kerndrahts (2);
Einbauen des Schraubenfederkörpers (3) **durch** Einführen des Schraubenfederkörpers (3) zu dem distalen Endabschnitt (21) des Kerndrahts (2), um um den Kerndraht (2) positioniert zu werden;
Bilden eines schweißgehärteten Abschnitts (413) an einem geschweißten Abschnitt zwischen den distalen Endspitzen des Kerndrahts (2) und dem Schraubenfederkörper (3) **durch** Schmelzen eines Schweißelements (4) als eutektische Legierung mit einer Schmelztemperatur von 180°C - 495°C, einschließlich einer eutektischen Legierung mit einer Schmelztemperatur von 180°C - 525°C, wenn der Austenitedelstahldraht des Kerndrahts (2) Molybdän als Bestandteil aufweist;
Bilden eines kleineren schweißgehärteten Abschnitts (412) **durch** Abtrennen des schweißgehärteten Abschnitts (413) bei einer vorbestimmten Länge von einem distalen Spitzenabschnitt des schweißgehärteten Abschnitts (413); und
Vorsehen eines kopfnächsten Abschnitts (411) **durch** das gleiche Schweißelement (4) oder die gleiche Art von Schweißelement, aus dem der kleinere schweißgehärtete Abschnitt (412) hergestellt ist, und Anbringen des kopfnächsten Abschnitts (411) integral mit einem distalen Spitzenabschnitt des kleineren schweißgehärteten Abschnitts (412), um den Kopfstopfen (41) zu bilden;
wobei das gesamte Querschnittreduktionsverhältnis eine Reduktionsrate R, ausgedrückt **durch** R = (S1 - S2)/S1 bedeutet, wobei S1 eine Querschnittfläche bezüglich eines ursprünglichen Durchmessers des mit dem Festlösungsvorgang behandelten Kerndrahts (2) ist, bevor der Kerndraht (2) gezogen wird, und S2 eine sich ergebende Querschnittfläche bezüglich eines fertigen Durchmessers des Kerndrahts (2) ist, nachdem der Kerndraht (2) schließlich während des Ziehprozesses gezogen wurde.

2. Verfahren zum Herstellen des medizinischen Führungsdrahts (1) nach Anspruch 1, wobei nach dem Schleifen des distalen Endabschnitts (21) des Kerndrahts (2) ein distales Ende des Kerndrahts (2) teilweise unter einer Temperatur von 180°C - 495°C zumindest an einem Abschnitt, in dem der Kopfstopfen (41) ausgebildet ist, einschließlich einer Temperatur von 180°C -525°C, wenn der Austenitedelstahldraht des Kerndrahts (2) Molybdän als Bestandteil aufweist, wärmebehandelt wird, und ein Schweißelement (4) geschmolzen wird, um über eine Außenfläche des Kerndrahts (2) um eine vorbestimmte Länge desselben gegossen zu werden, um eine Filmschicht (44) zu bilden, so dass das distale Ende des Kerndrahts (2), der kleinere schweißgehärtete Abschnitt (412) und der kopfnächste Abschnitt (411) vereint werden, um den Kopfstopfen (41) zu bilden, wobei das Schweißelement (4) das gleiche Schweißelement oder die gleiche Art von Schweißelement, das verwendet wird, wenn der Kopfstopfen (41) gebildet wird, ist.

## Revendications

1. Procédé pour fabriquer un fil guide médical (1) ayant une tige d'armature (2) formée par un élément allongé souple, un corps de ressort hélicoïdal (3) inséré dans une partie d'extrémité distale (21) de ladite tige d'armature (2) à placer autour de ladite tige d'armature (2), et un bouchon de tête (41) prévu au niveau des pointes d'extrémité distales à la fois de ladite tige d'armature (2) et dudit corps de ressort hélicoïdal (3) au moyen d'un élément de soudage (4), **caractérisé par** les étapes consistant à :
étirer ladite tige d'armature (2) qui est réalisée avec un fil d'acier inoxydable austénitique traité avec une procédure à base de solution solide jusqu'à ce que la totalité du rapport de réduction transversale de ladite tige d'armature (2) atteigne 90% - 99% ;
meuler ladite partie d'extrémité distale (21) de ladite tige d'armature (2) ;
assembler ledit corps de ressort hélicoïdal (3) en insérant ledit corps de ressort hélicoïdal (3) dans ladite partie d'extrémité distale (21) de ladite tige d'armature (2) à placer autour de ladite tige d'armature (2) ;
former une partie trempée au chalumeau (413) au niveau d'une partie soudée entre lesdites pointes d'extrémité distales de ladite tige d'armature (2) et dudit corps de ressort hélicoïdal (3) en faisant fondre un élément de soudage (4) en tant qu'alliage eutectique ayant une température de fusion de l'ordre de 180°C à 495°C comprenant un alliage eutectique ayant une température de fusion de l'ordre de 180°C à 525°C lorsque ledit fil d'acier inoxydable austénitique de ladite tige d'armature (2) comporte du molybdène en tant que composant ;
former une partie mineure trempée au chalumeau (412) en coupant ladite partie trempée au chalumeau (413) à une longueur prédéterminée à partir d'une partie de pointe distale de ladite partie trempée au chalumeau (413) ; et
prévoir une partie située le plus à proximité de la tête (411) avec le même ou le même type d'élément de soudage (4) à partir duquel ladite partie mineure trempée au chalumeau (412) a été réalisée, et fixer ladite partie située le plus près de la tête (411) de manière solidaire avec une partie de pointe distale de ladite partie mineure trempée au chalumeau (412) afin de former ledit bouchon de tête (41) ;
ladite totalité du rapport de réduction transversale signifiant un taux de réduction R exprimé par R = (S1 - S2) / S1, S1 étant une surface transversale concernant un diamètre d'origine de ladite tige d'armature (2) traitée avec ladite procédure à base de solution solide avant que ladite tige d'armature (2) ne soit étirée, et S2 étant une surface transversale résultante concernant un diamètre fini de ladite tige d'armature (2) après que ladite tige d'armature (2) a été finalement étirée pendant ledit procédé d'étirage.

2. Procédé pour fabriquer ledit fil guide médical (1) selon la revendication 1, dans lequel après avoir meulé ladite partie d'extrémité distale (21) de ladite tige d'armature (2), une extrémité distale de ladite tige d'armature (2) est traitée thermiquement partiellement à une température de l'ordre de 180°C à 495°C au niveau d'au moins une partie dans laquelle ledit bouchon de tête (41) est formé, comprenant une température de l'ordre de 180°C à 525°C lorsque ladite tige d'acier inoxydable austénitique de ladite tige d'armature (2) a du molybdène en tant que composant, et un élément de soudage (4) est fondu pour être déversé sur une surface externe de ladite tige d'armature (2) selon sa longueur prédéterminée afin de former une couche de film (44), de sorte que ladite extrémité distale de ladite tige d'armature (2), ladite partie mineure trempée au chalumeau (412) et ladite partie située le plus à proximité de la tête (411) sont assemblées pour former ledit bouchon de tête (41), ledit élément de soudage (4) étant le même ou du même type qu'un élément de soudage, tel qu'utilisé lorsque ledit bouchon de tête (41) est formé.
